# EUROPÄISCHE PATENTSCHRIFT

(11) **EP 0 702 228 B1**
(45) Veröffentlichungstag und Bekanntmachung des Hinweises auf die Patenterteilung: **24.11.1999**
(21) Anmeldenummer: 95890161.3
(22) Anmeldetag: 11.09.1995
(51) Int. Cl.: G01N 27/327, C12Q 1/00

(54) **Planarer Sensor zum Erfassen eines chemischen Parameters einer Probe**
Planar sensor for measuring a chemical parameter of a sample
Capteur planaire de mesure d'un paramètre chimique d'un échantillon

(30) Priorität: 14.09.1994 AT 176094
(43) Veröffentlichungstag der Anmeldung: 20.03.1996
(73) Patentinhaber: AVL Medical Instruments AG, 8207 Schaffhausen (CH)
(72) Erfinder: Schaffar, Bernhard Mag. Dr., A-8045 Graz (AT); Kontschieder, Heinz, Dipl.-Ing., A-8010 Graz (AT); Dolezal, Andreas, A-8045 Graz (AT); Ritter, Christoph, Dr., A-8045 Graz (AT)
(74) Vertreter: Krause, Walter, Dr. Dipl.-Ing.

(56) Entgegenhaltungen:
- EP-A- 0 206 218
- EP-A- 0 354 204
- EP-A- 0 476 980
- EP-A- 0 609 760
- US-A- 4 894 137
- US-A- 5 326 449
- PATENT ABSTRACTS OF JAPAN vol. 012, no. 443 (P-790), 22.November 1988 & JP 63 172951 A (FUJITSU LTD), 16.Juli 1988,

## Beschreibung

Die Erfindung betrifft einen planaren Sensor zum Erfassen eines chemischen Parameters einer Probe, bestehend aus einem Träger mit zumindest teilweise planer Oberfläche, welche zumindest einen potentiometrischen oder amperometrischen sowie ggf. einen optischen Transducer und zumindest eine biologische Komponente aufweist, sowie mit einer probenseitig angebrachten Deckmembran, wobei der Transducer und die biologische Komponente in zumindest einem Teilbereich auf der Oberfläche des Trägers als sogenannter Sensorspot vorliegen und die Deckmembran rund um den Sensorspot mit dem Träger verschweißt ist.

Derartige Sensoren finden vor allem Verwendung in der Medizintechnik sowie in der Biotechnologie und Lebensmittelanalytik. Die biologische Komponente des Sensors kann beispielsweise ein Mikroorganismus, ein Enzym, ein Antikörper bzw. Antigen sein oder auch DNA/RNA. Unter Transducer bzw. Transducerschicht ist die Summe jener Elemente zu verstehen, welche die vorerst stoffliche bzw. materielle Information des biologischen Systems in ein Stromsignal (amperometrisch), Spannungssignal (potentiometrisch) oder Lichtsignal (photometrisch) umwandelt. Ein optischer Transducer kann beispielsweise einen Fluoreszenzfarbstoff aufweisen, dessen optische Eigenschaften sich beispielsweise abhängig von der O₂-Konzentration oder des pH-Wertes ändern. Bei potentiometrischen Sensoren kann die ionenselektive Membran zwischen Leiterbahn und Enzymschicht als potentiometrischer Transducer bezeichnet werden.

Die probenseitig angebrachte Deckmembran dient einerseits zur Diffusionslimitierung, wenn die zu bestimmende Probenkonzentration den analytischen Meßbereich des Sensors übersteigt und andererseits zum mechanischen Schutz der biologischen Komponente bzw. zur Herstellung einer gewissen Biokompatibilität.

Die Deckmembran derartiger Sensoren ist mit Poren versehen bzw. aus porösem Material gefertigt, wobei je nach Meßgebiet und Anwendung Porendurchmesser von 100Å bis 5000Å möglich sind. Die Poren in der Deckmembran lassen einen Kontakt der zu messenden Substanz mit der unter der Deckmembran liegenden biologischen Komponente zu. Beim Anbringen der Deckmembran ist bei derartigen Sensoren darauf zu achten, daß die empfindliche biologische Komponente nicht zerstört oder in ihrer Wirkung beeinträchtigt wird.

Eine weitere Aufgabe der Deckmembran besteht darin, die vorgefertigten Sensorspots, welche besonders kostengünstig größflächig hergestellt werden können, auf der Oberfläche bzw. in Ausnehmungen eines Trägers festzuhalten.

In "Sensors and Actuators B", 15 (1993), 113 (BILITEVSKY et al.) wird eine Enzymelektrode zur Bestimmung von Kohlehydraten in Lebensmitteln beschrieben. Als Deckmembran wird hier eine Polycarbonatmembran auf einen Planarsensor geklebt. Zuerst wird ein mittels Siebdruck aufbringbarer Kleber auf einem Träger aufgebracht, danach wird die Deckmembran mittels angelegtem Vakuum in den Kleber gepreßt. Damit die Luft zwischen Sensoroberfläche und Polycarbonatmembran entweichen kann, sind in den Träger vorher Löcher zu bohren. Danach wird der Kleber für fünf Minuten bei 120° C gehärtet, wobei in einer speziellen Vorrichtung der eigentliche Sensorspot gekühlt werden muß. Nachteilig bei diesem Planarsensor sind die vielen Arbeitsschritte bei der Herstellung sowie vor allem das Aufbringen und Härten des Klebers bei relativ hohen Temperaturen, bei welchen eine aufwendige Kühlung der Sensorspots erforderlich ist, da die biologische Komponente in der Regel derartig hohe Temperaturen nicht verträgt. Als weiterer Nachteil ist zu erwähnen, daß potentiell austretende Monomere bzw. Oligomere bzw. Lösungsmittelmoleküle aus der Kleberschicht vor deren Aushärtung die biologische Komponente beinträchtigen könnten.

Als weiteres Beispiel für die Befestigung der Deckmembran auf der Oberfläche derartiger Biosensoren ist die US-A 4 073 713 zu nennen. Hier wird eine Polycarbonatmembran mittels O-Ring an die Sensoroberfläche gedrückt. An der Polycarbonatmembran ist auf der Sensorseite das Enzym immobilisiert bzw. es kann auch eine zweite Membran befestigt sein. Diese Befestigungstechnik läßt sich nicht einfach auf Planarsensoren anwenden. Weiters ist auch die für viele Anwendungsgebiete wünschenswerte Miniaturisierung der Sensoren nicht möglich. Halb- bzw. vollautomatische Fertigungsprozesse werden erschwert und verteuern sich.

Aus der US-A 5 326 449 ist schließlich ein Sensor mit einer Mehrschichtmembran (Composite-Membrane) bekannt, welche sich unter anderem zur Glucosemessung eignet. Die Mehrschichtmembran dieses amperometrischen Sensors besteht aus einer porösen Membran, in welche eine biologisch aktive Komponente, z.B. ein Protein, immobilisiert ist und aus zumindest einer weiteren Membran. Die Mehrschichtmembran kann gemäß einem ersten Ausführungsbeispiel durch Ultraschallschweißen mit der Trägerschicht verbunden sein. In einer weiteren Ausführungsvariante der US-A 5 326 449 wird vorgeschlagen, eine über die eigentliche Sensormembran hinausragende Deckmembran (Protecting Membrane) vorzusehen, welche außerhalb der eigentlichen Sensormembran mit der Trägerschicht verschweißt ist. Die Trägerschicht weist eine Öffnung auf, welche ein elektrisch leitendes Element zur Signalableitung durchsetzt.

Aus der US-A 4,894,137 ist eine Enzymelektrode bekannt, deren Leiterbahnen mittels Photolitographie auf die Oberfläche der Trägerschicht aufgebracht werden. Die Leiterbahnen verbinden die Arbeitselektrode bzw. die Referenzelektrode mit den Zu- und Ableitungen, welche an einem Ende der Leiterbahnen angelötet und mit einer Isolierschicht vergossen sind. Die Enzymelektrode weist eine Interferenzsperrschicht, eine Enzymschicht und eine Deckmembran auf, welche als Lösung aufgebracht werden.

Aufgabe der vorliegenden Erfindung ist es, einen planaren Biosensor vorzuschlagen, bei welchem die probenseitige Anbringung der Deckmembran rasch und effizient erfolgt, wobei das Herstellungsverfahren eine einfache, halb- bzw. vollautomatische Massenfertigung derartiger Sensoren zulassen soll.

Diese Aufgabe wird erfindungsgemäß dadurch gelöst, daß jene Sensorspots, die einen potentiometrischen oder amperometrischen Transducer aufweisen, mit einer an der planen Oberfläche des Trägers angebrachten Leiterbahn kontaktiert sind, wobei die Verschweißung der Deckmembran im Bereich der von den Sensorspots wegführenden Leiterbahnen unterbrochen ist.

Entscheidende Vorteile bei der automatisierten Herstellung derartiger planarer Sensoren können somit dadurch erzielt werden, daß sich die gesamte Sensoranordnung inklusive Ableitbahnen auf einer Seite des Trägers befindet und keinerlei Bohrungen bzw. Durchkontaktierungen durch den Träger notwendig sind. Alle wesentlichen Komponenten wie Leiterbahnen, Referenz- und Gegenelektroden sowie Arbeitselektroden lassen sich beispielsweise mittels Siebdruck bzw. Dispensiertechnik aufbringen. Überraschenderweise konnte überdies in entsprechenden Versuchen dargelegt werden, daß durch einen Heißprägestempel, welcher an den Positionen, in denen die Sensorspots vorliegen bzw. die Leiterbahnen von den Sensorspots wegführen, Ausnehmungen aufweist, einerseits die Hitzebelastung auch von sehr temperaturempfindlichen biologischen Komponenten, wie etwa dem Enzym Lactatoxidase derart niedrig gehalten werden kann, daß eine zusätzliche Kühlung des Sensorspots entfallen kann und andererseits eine Beschädigung der Leiterbahnen ausgeschlossen werden kann. Dabei kann der Heißprägestempel - beispielsweise bei der Verschweißung von Polycarbonat - ca. 200° C aufweisen und die Prägedauer einige Sekunden andauern. Durch das Anlegen eines höheren Druckes kann allerdings sowohl die Verweildauer des Prägestempels als auch die Prägetemperatur erniedrigt werden. Weiters kann gegebenenfalls die Verschweißung auch nur an einzelnen Punkten oder Flächenabschnitten geschehen, was eine weitere Reduktion der thermischen Belastung bewirkt. Als weiterer Vorteil sei angeführt, daß die Prägestempel so ausgeführt sein können, daß ein Selbstspannen der Deckmembran erfolgt, was insbesonders für Sensoren mit gewünschtem raschem Ansprechverhalten vorteilhaft ist. Durch Einhaltung der Parameter Temperatur, Anpreßdruck und Schweißdauer kann der Vorgang automatisiert und hoch reproduzierbar durchgeführt werden. Der erfindungsgemäße Sensor weist keine Klebestellen auf, sodaß alle Nachteile im Zusammenhang mit der Aufbringung der Klebechemikalien der Aushärtung der Kleber sowie die Änderungen der Diffusionseigenschaften der Deckmembran durch die Kleber entfallen.

Ein erfindungsgemäßes Herstellungsverfahren eines planaren Sensors zum Erfassen eines chemischen Parameters einer Probe ist beispielsweise durch folgende Schritte gekennzeichnet.
a) Aufbringen einer Leiterbahn auf zumindest einen Teilbereich der Oberfläche eines Trägers,
b) Aufbringen einer potentiometrischen oder amperometrischen Transducerschicht auf einen Endbereich der Leiterbahn
c) Aufbringen zumindest einer biologischen Komponente auf die Transducerschicht,
d) Bedecken zumindest der Transducerschicht und eines Umgebungsbereiches mit einer mit dem Träger verschweißbaren Deckmembran,
e) Verschweißen der Deckmembran mit dem Träger in den von der Transducerschicht und der biologischen Komponente nicht beanspruchten Bereichen, wobei die Verschweißung im Bereich der Leiterbahn unterbrochen wird.

Anstelle der Punkte b) und c) ist es in einer Ausführungsvariante jedoch auch möglich, ein polymeres Matrixmaterial auf einen Endbereich der Leiterbahn aufzubringen und den potentiometrischen oder amperometrischen Transducer und die biologische Komponente auf bzw. im polymeren Matrixmaterial zu immobilisieren, d. h. Transducer-Schicht und biologische Komponente werden als eine Schicht aufgebracht.

Zur Automatisierung des Verfahrens kann die Deckmembran von einem Band abgerollt, über die Oberfläche des Trägers gespannt und mit Hilfe eines Heißprägestempels mit dem Träger (thermisch) verschweißt werden. Dabei kann erfindungsgemäß eine flächige oder punktförmige Verschweißung durchgeführt werden.

Es wäre gemäß der vorliegenden Erfindung jedoch auch denkbar, die Deckmembran mittels Laserlicht mit dem Träger thermisch zu verschweißen.

Die verschweißbare Deckmembran kann aus einem thermoplastischen Kunststoff, vorzugsweise aus Polycarbonat, Polyurethan, Polysulfon oder Celluloseacetat, der Träger zumindest in den mit der Deckmembran verschweißten Bereich aus einem thermoplastischen Kunststoff, vorzugsweise aus Polycarbonat, Polysulfon, PVC oder Plexiglas (PMMA) bestehen.

Neben der Verwendung einer beliebigen herkömmlichen Referenzelektrode (z.B. Kalomelelektrode) kann bei einer potentiometrischen oder amperometrischen Meßanordnung zumindest ein Sensorspot als Arbeitselektrode ausgebildet sein und weiters ggf. zumindest eine Referenz- und/oder Gegenelektrode an der Oberfläche des Trägers angeordnet sein.

Zur Erstellung exakter Kontroll- oder Referenzmeßpunkte ist es weiters erfindungsgemäß von Vorteil, wenn zumindest zwei Sensorspots vorgesehen sind, wobei ein Sensorspot eine aktive bio logische Komponente enthält und der andere dieselbe, inaktivierte Komponente aufweist. Dadurch wird eine Kompensationselektrode realisiert.

Bei einer derartigen Anordnung besteht gemäß der Erfindung die Möglichkeit, daß die Deckmembran die Referenz- und/oder Gegenelektrode bedeckt und rund um diese Elektroden mit dem Träger verschweißt ist, wobei die Verschweißung im Bereich der von den Elektroden wegführenden Leiterbahnen unterbrochen ist. Die Deckmembran bietet dabei den Vorteil eines mechanischen Schutzes der Elektrodenoberflächen, z. B. einen Schutz vor Belegung mit roten Blutkörperchen.

In einer weiteren vorteilhaften Ausführungsvariante der Erfindung ist vorgesehen, daß ein zusätzlicher Sensorspot mit einem optischen Transducer vorgesehen ist, welcher eine Indikatorschicht mit einem optischen Indikator sowie zumindest eine Schicht mit der biologischen Komponente aufweist.

Erfindungsgemäß ist es schließlich auch möglich, daß ein zusätzlicher Sensorspot mit einer Polymermatrix vorgesehen ist, in bzw. auf welche der optische Indikator und die biologische Komponente chemisch oder physikalisch immobilisiert vorliegen.

Die Erfindung wird im folgenden anhand von Zeichnungen näher erläutert. Es zeigen Fig. 1 eine schematische Darstellung einer Apparatur zum Verschweißen der Deckmembran mit dem Träger, die Fig. 2a bis 8a erfindungsgemäße Planarsensoren, die Fig. 2b bis 8b die jeweils erforderlichen Heißprägestempel bzw. Sonotroden, sowie die Fig. 9 bis 21 schematische Schnittdarstellungen verschiedener Ausführungsvarianten der erfindungsgemäßen Planarsensoren.

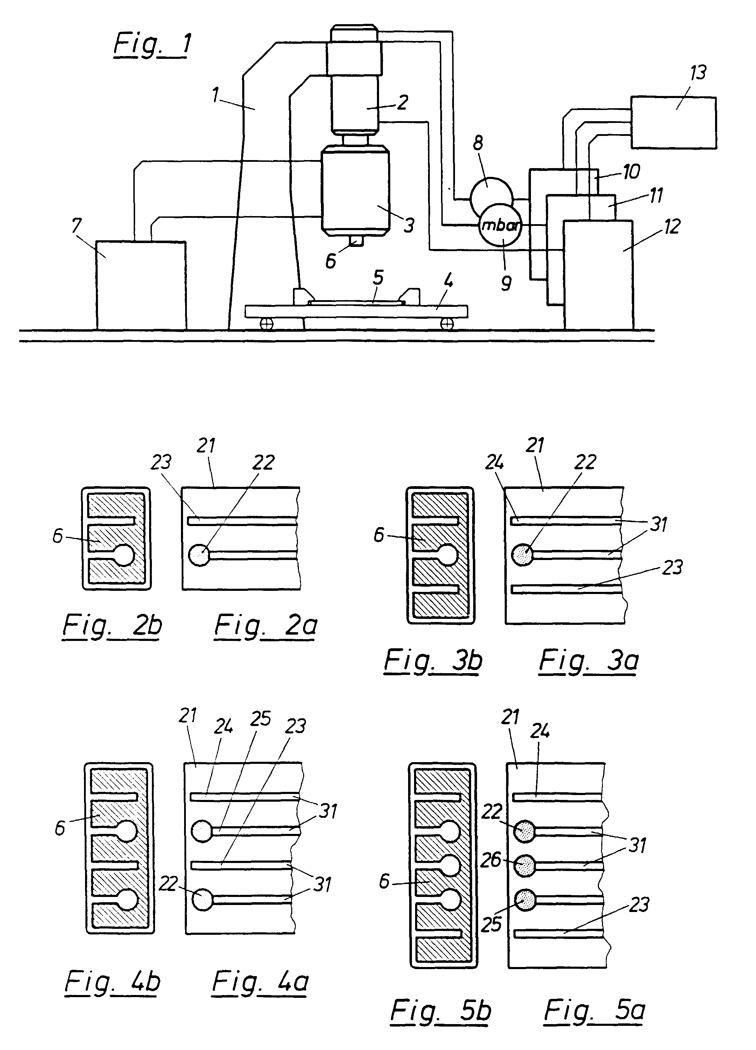

Fig. 1 beschreibt eine Vorrichtung zum Verschweißen einer Deckmembran mit der Oberfläche eines Trägers eines planaren Sensors. Die Vorrichtung weist eine Kniehebelpresse 1 auf, mit deren Druckluftzylinder 2 ein Thermostatisierblock 3 in Richtung eines in X- und Y-Richtung verstellbaren Tisches 4 abgesenkt werden kann. Der Tisch 4 weist eine Halteeinrichtung 5 für den Träger des planaren Sensors auf. An der der Halteeinrichtung 5 zugewandten Seite des Thermostatisierblockes 3 ist ein Heißprägestempel 6 angeordnet, dessen Prägeprofil anhand der folgenden Ausführungsvarianten des planaren Sensors näher beschrieben wird. Weiter weist der Thermostatisierblock 3 einen Thermostat 7 mit Temperatursteuerung auf. Als zusätzliche Einrichtungen sind Manometer 8 und 9 zur Haltedrucksteuerung bzw. zur Schweißdrucksteuerung erkennbar sowie schematisch dargestellte Zeitgeber 10 bis 12 zur Steuerung der Schweißzeit sowie zur Auf- und Abbewegung des Prägestempels 6. Mit 13 ist eine zentrale Steuereinheit zur Koordinierung der Zeitgeber 10 bis 12 dargestellt.

Eine Vorrichtung zum Verschweißen mittels Ultraschall kann ähnlich ausgeführt sein und anstelle des Prägestempels 6 eine Sonotrode aufweisen.

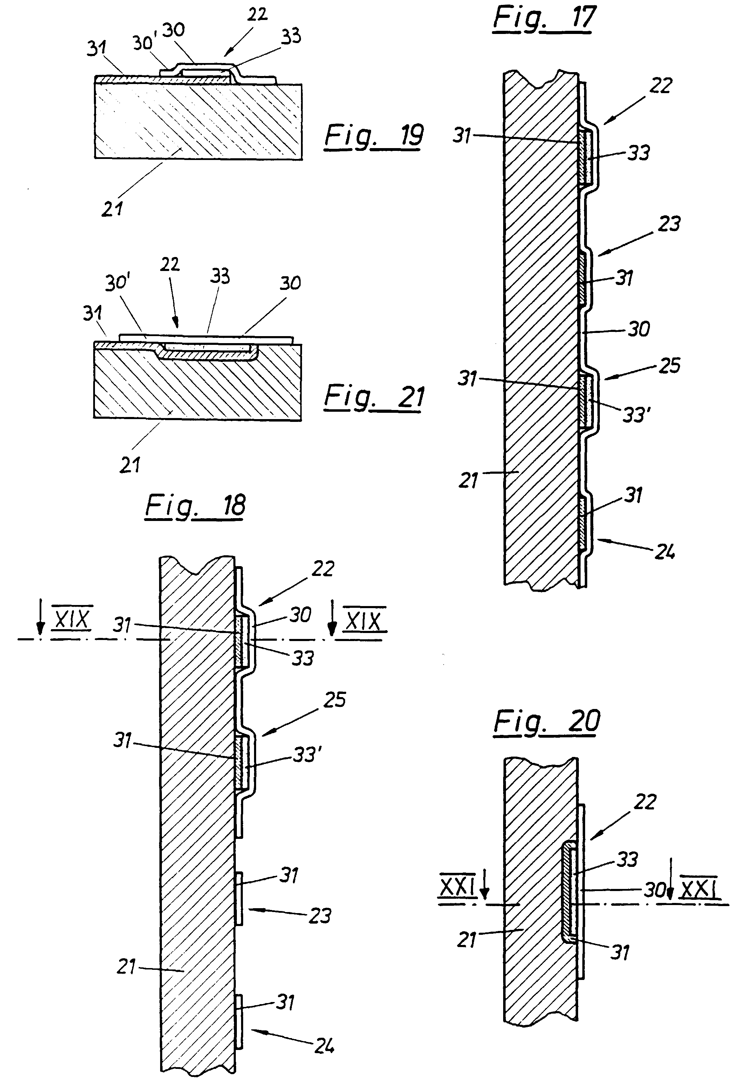

Die erfindungsgemäßen planaren Sensoren der Fig. 2a bis 9a sind in Draufsicht bei weggelassener Deckschicht dargestellt, wobei jeweils der Träger mit 21 und die verschiedenen Varianten der Heißprägestempel in den zugehörigen Fig. 2b bis 9b mit 6 gekennzeichnet sind. Der Heißprägestempel 6 ist jeweils im Bereich seiner Ausnehmungen für die einzelnen Sensorspots und Leiterbahnen geschnitten dargestellt, sodaß die schraffierte Fläche der eigentlichen Prägefläche des jeweiligen Stempels entspricht. Falls eine Ultraschallschweißtechnik angewendet wird, stellt die schraffierte Fläche die aktive Oberfläche der Sonotrode dar. Im Bereich der von den Sensorspots wegführenden Leiterbahnen 31 ist die Prägefläche und damit auch die Verschweißung der Membran mit dem Träger 21 unterbrochen (siehe Fig. 19 u. 21). Es sind jedoch auch Varianten von Heißprägestempeln denkbar, in denen auch Ausnehmungen zwischen verschiedenen Sensorspots vorliegen oder nur punktuell um die Sensorspots geprägt oder geschweißt wird.

In den Fig. 2a und 3a wird jeweils ein amperometrischer Biosensor dargestellt, wobei die Arbeitselektrode mit 22 und die Referenzelektrode mit 23 bezeichnet ist. Der Biosensor in Fig. 3a weist zusätzlich eine Gegenelektrode 24 auf.

Fig. 4a zeigt einen amperometrischen Biosensor in einer Zwei-Sensortechnik. Neben einer Arbeitselektrode 22 mit einem aktiven Enzym befindet sich noch eine Vergleichselektrode 25 mit einem inaktiven Enzym auf der Oberfläche des Trägers 21. Fig. 5a zeigt einen Träger mit zwei amperometrischen Biosensoren 22 und 26, welche über gemeinsame Referenz-, Gegen- und Vergleichelektroden 23 bis 25 verfügen. Die Arbeitselektrode 22 trägt ein erstes aktives Enzym und die weitere Arbeitselektrode 26 ein zweites aktives Enzym, wobei die Signale beider Elektroden als Differenzsignal zur Vergleichselektrode 25 gewonnen werden können.

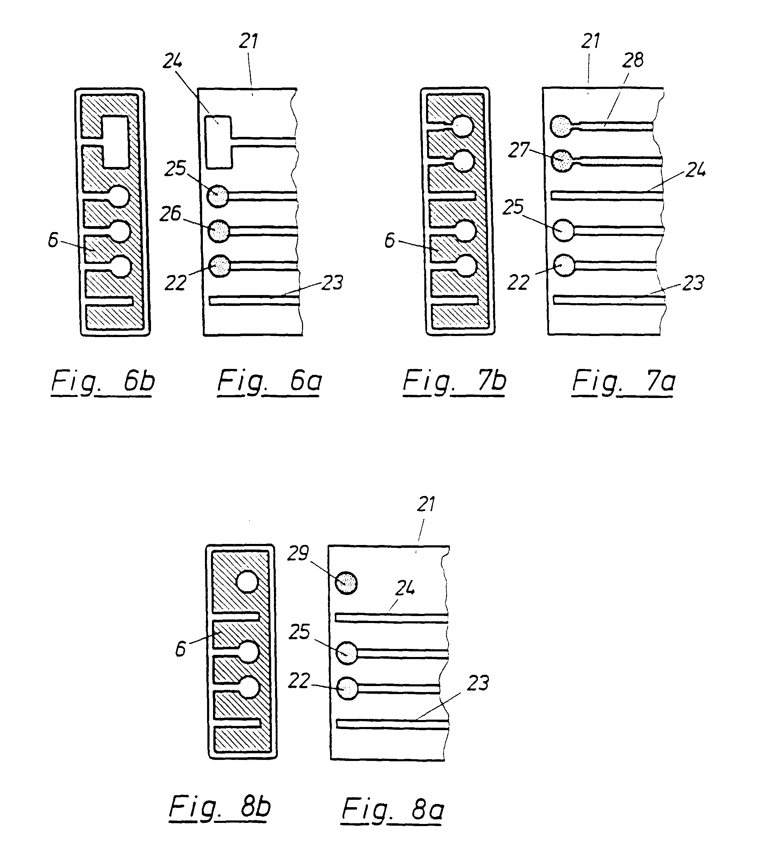

Fig. 6a zeigt eine ähnliche Anordnung wie Fig. 5a mit vergrößerter gemeinsamer Gegenelektrode 24.

Fig. 7a zeigt auf einem Träger die Kombination von amperometrischen und potentiometrischen Biosensoren. Neben der bereits bei den früheren Ausführungsvarianten beschriebenen amperometrischen Arbeitselektrode 22 mit einem ersten aktiven Enzym und den dazugehörigen Referenz-, Gegen- und Vergleichselektroden 23 bis 25 weist dieser Träger einen potentiometrischen Biosensor 27 sowie eine potentiometrische Referenzelektrode 28 auf.

In der Ausführungsvariante nach Fig. 8a ist neben einer amperometrischen Arbeitselektrode 22 mit einem aktiven Enzym und den erforderlichen Referenz-, Gegen- und Vergleichselektroden 23 bis 25 ein Biosensor 29 vorgesehen, welcher einen z. B. durch den Sauerstoffgehalt in seinen optischen Eigenschaften veränderbaren Farbstoff enthält.

Die genannten Elektroden 22, 25, 26, 27, 28 sowie die Optode 29 der beschriebenen Ausführungsvarianten liegen als sogenannte Sensorspots auf der im wesentlichen ebenen oder leicht gekrümmten Oberfläche des Trägers vor, bzw. werden vorgefertigt in Ausnehmungen auf der Trägeroberfläche eingelegt.

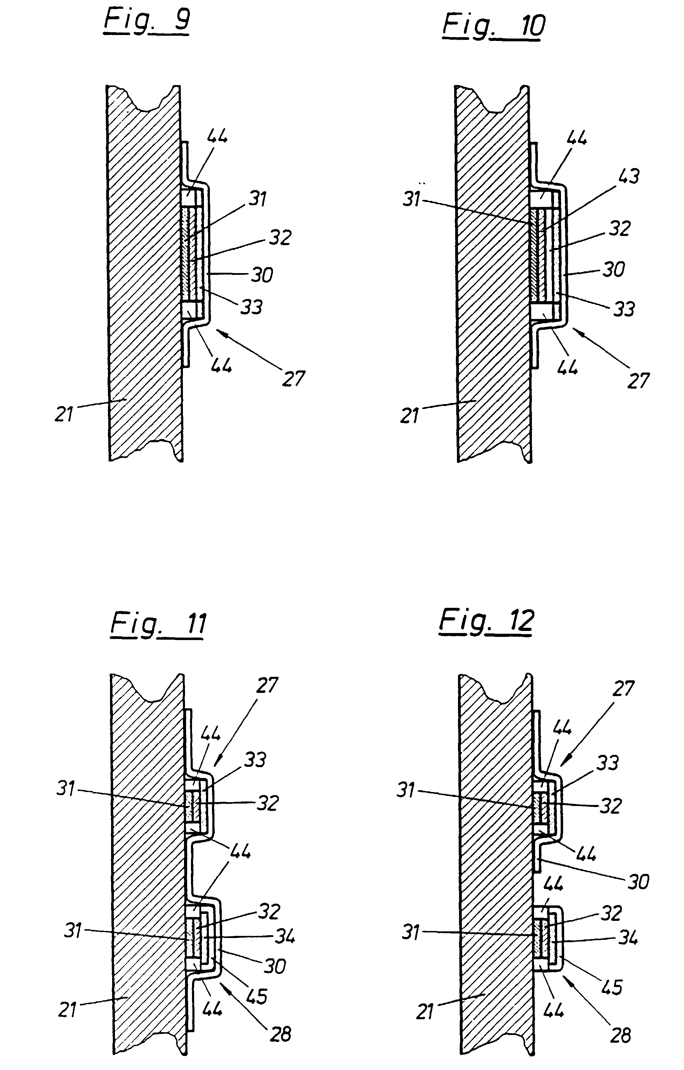

Der Aufbau der unterschiedlichen Sensorspots bzw. auch der Referenz- und Gegenelektroden wird in den Schnittdarstellungen Fig. 9 bis Fig. 21 näher erläutert.

In den Fig. 9 bis 12 befindet sich als Beispiel für einen potentiometrischen Biosensor auf einem Träger 21 ein Sensorspot 27 (Arbeitselektrode mit einem aktiven Enzym), welcher aus einer Leiterbahn 31 einer ionenselektiven Membran 32 und einer Enzymschicht 33 besteht. Die Deckmembran 30 ist rund um den Sensorspot 27 mit dem Träger 21 verschweißt. Rund um den Sensorspot und über der Leiterbahn befindet sich eine Isolierschicht 44, z.B. aus Polyurethan. Die Ausführung nach Fig. 10 unterscheidet sich von jener nach Fig. 9 nur dadurch, daß sich zwischen der Leiterbahn 31 und der ionenselektiven Membran 32 eine Kontaktschicht 43 befindet. Bei den Ausführungsvarianten gemäß Fig. 11 und Fig. 12 befindet sich auf dem Sensorplättchen zusätzlich eine potentiometrische Referenzelektrode 28, welche beginnend auf der Oberfläche des Trägers beispielsweise aus einer Leiterbahn 31 einer ionenselektiven Membran 32, aus einer Hydrogel-Elektrolytschicht 34 sowie einer Enzymschicht 33 besteht. In der Ausführung nach Fig. 11 befindet sich die potentiometrische Referenzelektrode 28 ebenfalls unter der Deckmembran 30, in der Ausführung nach Fig. 12 außerhalb der Deckmembran.

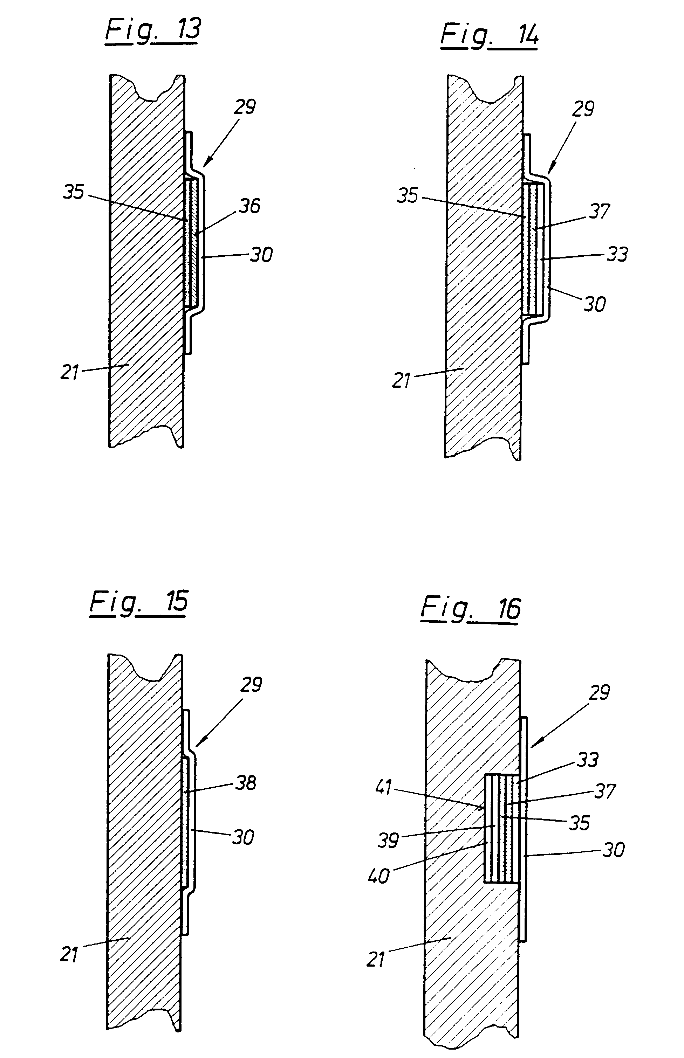

In den Ausführungsvarianten gemäß Fig. 13 bis 16 befinden sich als Beispiel für einen optischen Biosensor auf optisch durchlässigen Trägern 21 Optoden 29 von unterschiedlichem Aufbau. Die dargestellten Optoden sind mit amperometrischen oder potentiometrischen Sensoren (nicht dargestellt) auf einem gemeinsamen Träger integriert. Bei der Ausführung nach Fig. 13 weist die Optode eine Indikatorschicht 35 mit einer optischen Indikatorsubstanz sowie eine darüberliegende Enzymschicht 36 mit einer Pigmentierung zur optischen Isolierung der Indikatorschicht auf. Auch hier ist die Deckschicht 30 rund um die Optode 29 mit dem Träger 21 verschweißt. Bei der Ausführung nach Fig. 14 liegt die optische Isolierschicht 37 außerhalb der Enzymschicht 33. Eine besonders einfache Ausführungsvariante stellt die Fig. 15 dar, wo die Optode 29 eine Polymermatrix 38 aufweist, in welcher bzw. auf welche der optische Indikator und die biologische Komponente (beispielsweise ein Enzym) chemisch oder physikalisch immobilisiert vorliegen. Bei der Ausführungsvariante nach Fig. 16 liegt eine Optode 29 gemäß Fig. 14 (Indikatorschicht 35, Isolierschicht 37, Enzymschicht 33) auf einer transparenten Folie 39 vor, welche mit einer optischen Ankopplung 40 in eine Ausnehmung 41 des transparenten Trägers 21 eingesetzt ist. Die ganze Anordnung wird mit einer ebenen Deckmembran 30 verschlossen und rund um den Sensorspot verschweißt.

Die Ausführungen nach Fig. 17 bis Fig. 21 zeigen planare amperometrische Biosensoren, wobei bei den Varianten Fig. 17 und Fig. 18 zusätzlich zu den Arbeitselektroden 22 Referenz- und Gegenelektroden 23, 24 am Träger angeordnet sind. Die Fig. 19 zeigt einen Schnitt entlang der Linie XIX-XIX in Fig. 18 sowie die Fig. 21 einen Schnitt entlang der Linie XXI-XXI in Fig. 20 jeweils im Bereich der Arbeitselektrode 22. Die Verschweißung der Deckmembran 30 ist jeweils im Bereich 30' der von den Sensorspots wegführenden Leiterbahn 31 unterbrochen.

Bei den Ausführungsvarianten nach Fig. 17 und Fig. 18 befindet sich auf den Trägern jeweils eine Arbeitselektrode 22 und eine Vergleichselektrode 25. Die Arbeitselektroden weisen auf einer Leiterbahn 31 eine aktive Enzymschicht 33 auf. In den Sensorspots der Vergleichselektroden 25 befindet sich eine inaktive Enzymschicht 33'. In der Fig. 17 befinden sich alle Elektroden unter der Deckmembran 30, bei der Fig. 18 nur die Arbeits- und die Vergleichselektrode 22 und 25. Schließlich zeigt die Ausführungsvariante nach Fig. 20 eine in den Träger 21 versenkte Arbeitselektrode 22, wobei die Leiterbahn 31 die Enzymschicht 33 umfaßt und von einer ebenen Deckmembran 30 verschlossen wird.

Im folgenden werden einige Beispiele zur Herstellung von Biosensoren mit verschweißter Deckmembran angegeben.

### Beispiel A:

Herstellung eines amperometrischen Glucosebiosensors mit Kompensationselektrode mit einem H₂O₂-Transducer (Fig. 4a, 4b und 17).

Auf einem Trägerplättchen 21 aus Polycarbonat werden die Leiterbahnen 31 der Arbeitselektroden 22 und der Kompensationselektrode 25 mittels Siebdrucktechnik mit einer Gold- bzw. Platinpaste gedruckt. Die Leiterbahnen 31 der Referenz- 23 und Gegenelektrode 24 werden mittels Siebdrucktechnik mit einer Ag/AgCl Paste gedruckt. Auf die Leiterbahn wird bei der Arbeitselektrode 22 eine Paste auf Graphit bzw. Platinbasis mit dem aktiven Enzym Glucoseoxidase als Enzymschicht 33 (Fig. 17) mittels Siebdrucktechnik bzw. Dispensiertechnik aufgebracht. Gleichermaßen wird mit dem Sensorspot für die Kompensationselektrode 25 verfahren, außer, daß anstatt Glucoseoxidase Rinderserumalbumin oder durch Hitze inaktivierte Glucoseoxidase immobilisiert wird. Danach wird als Deckmembran 30 ein Streifen einer Polycarbonatfolie mit 300 Å Porenweite aufgelegt und mit einem Schweißstempel (Fig. 4b) bei 210° C für 3s bei 1,5 bar Haltedruck verschweißt.

### Beispiel B:

Herstellung eines potentiometrischen Harnstoffbiosensors auf der Basis einer Ammoniumelektrode (Fig. 10).

Auf einem Trägerplättchen 21 aus PMMA wird eine Leiterbahn 31 aus einer Silberpaste mittels Siebdrucktechnik aufgebracht. An der Probenseite wird mit gleicher Technik zuerst eine Festkörperkontaktschicht 43 mit einer Graphitpaste gedruckt und dann rund um den Sensorspot und über die Leiterbahn eine Schicht einer Isolationspaste aus Polyurethan 44. Direkt auf den Sensorspot wird anschließend ein Cocktail, bestehend aus PVC, Weichmacher (z.B. Dioctyladipat), dem Ionophor Nonactin und Kalium-p-Clorotetraphenylborat aus einer Lösung aus Tetrahydrofuran aufgetropft (dispensiert)und das Lösungsmittel verdampft. Auf die sich bildende Ammoniumionen selektive Membran 32 wird die biologische Komponente 33 in Form eines kovalent vernetzenden Hydrogels aus wäßriger Lösung aufgetropft. Danach wird eine Polycarbonatmembran 30 mit der Porenweite 500 Å aufgelegt und bei 185°C für 3s bei 1,5 bar Druck verschweißt. Der so hergestellte Sensor kann mit einer externen Referenzelektrode potentiometrisch betrieben werden.

### Beispiel C:

Herstellung eines fluoreszenzoptischen Biosensors für Ascorbat auf der Basis einer O₂-Optode (Fig. 16).

Auf eine transparente Folie 39 aus Polyethylenterephthalat wird die optische Indikatorschicht 35, der Fluoreszenzfarbstoff Decacyclen gelöst in einem essigsäurespaltenden einkomponentigen Silikon, großflächig aufgebracht. Zur optischen Isolierung 37 wird ein schwarz pigmentiertes einkomponentiges Silikon aufgebracht und darauf mittels Quervernetzung durch Glutardialdehyd das Enzym Ascorbatoxidase immobilisiert (33). Aus dieser Laminatfolie wird ein Sensorspot ausgestanzt und in die Ausnehmung 41 am Trägerplättchen 21 aus Polycarbonat mittels einem transparenten zweikomponentigen Silikon eingeklebt (optische Ankopplung 40), wobei die Dicke der Laminatfolie der Tiefe der Ausnehmung 41 im Trägerplättchen entspricht. Anschließend wird eine Polycarbonatmembran mit 500 Å Poren auf das Trägerplättchen rund um den optischen Sensorspot bei 210°C für 3s mit 1,5 bar Haltedruck verschweißt.

## Patentansprüche

1. Planarer Sensor zum Erfassen eines chemischen Parameters einer Probe, bestehend aus einem Träger (21) mit zumindest teilweise planer Oberfläche, welche zumindest einen potentiometrischen oder amperometrischen sowie ggf. einen optischen Transducer und zumindest eine biologische Komponente aufweist, sowie mit einer probenseitig angebrachten Deckmembran (30), wobei der Transducer und die biologische Komponente in zumindest einem Teilbereich auf der Oberfläche des Trägers (21) als sogenannter Sensorspot (22, 25, 26, 27, 28, 29) vorliegen und die Deckmembran (30) rund um den Sensorspot mit dem Träger (21) verschweißt ist, **dadurch gekennzeichnet**, daß jene Sensorspots (22, 25 26, 27), die einen potentiometrischen oder amperometrischen Transducer aufweisen, mit einer an der planen Oberfläche des Trägers (21) angebrachten Leiterbahn (31) kontaktiert sind, wobei die Verschweißung der Deckmembran (30) im Bereich der von den Sensorspots (22, 25, 26, 27) wegführenden Leiterbahnen (31) unterbrochen ist.

2. Sensor nach Anspruch 1 mit einer amperometrischen Meßanordung, **dadurch gekennzeichnet**, daß zumindest ein Sensorspot als Arbeitselektrode (22, 25, 26) ausgebildet ist und weiters zumindest eine Referenz- (23) und/oder Gegenelektrode (24) an der Oberfläche des Trägers (21) angeordnet ist.

3. Sensor nach Anspruch 2, **dadurch gekennzeichnet,** daß die Deckmembran (30) die Referenz- (23) und/oder Gegenelektrode (24) bedeckt und rund um diese Elektroden (23, 24) mit dem Träger (21) verschweißt ist, wobei die Verschweißung im Bereich der von den Elektroden (23, 24) wegführenden Leiterbahnen (31) unterbrochen ist.

4. Sensor nach Anspruch 1 mit einer potentiometrischen Meßanordnung, **dadurch gekennzeichnet**, daß zumindest ein Sensorspot als Arbeitselektrode (27) ausgebildet ist und ggf. zumindest eine Referenzelektrode (28) an der Oberfläche des Trägers (21) angeordnet ist.

5. Sensor nach einem der Ansprüche 2 bis 4, **dadurch gekennzeichnet**, daß ein zusätzlicher Sensorspot (29) mit einem optischen Transducer vorgesehen ist, welcher eine Indikatorschicht (35) mit einem optischen Indikator sowie zumindest eine Schicht (33, 36) mit der biologischen Komponente aufweist.

6. Sensor nach einem der Ansprüche 2 bis 4, **dadurch gekennzeichnet**, daß ein zusätzlicher Sensorspot (29) mit einer Polymermatrix (38) vorgesehen ist, in bzw. auf welche der optische Indikator und die biologische Komponente chemisch oder physikalisch immobilisiert vorliegen.

7. Sensor nach Anspruch 1 bis 6, **dadurch gekennzeichnet,** daß zumindest zwei Sensorspots vorgesehen sind, wobei ein Sensorspot als Arbeitselektrode (22, 25, 26, 27) eine aktive biologische Komponente enthält und der andere als Vergleichselektrode (25) dieselbe, inaktivierte Komponente aufweist.

8. Verfahren zur Herstellung eines planaren Sensors zum Erfassen eines chemischen Parameters einer Probe, **gekennzeichnet durch** folgende Schritte:
a) Aufbringen einer Leiterbahn auf zumindest einen Teilbereich der Oberfläche eines Trägers,
b) Aufbringen einer potentiometrischen oder ampero-metrischen Transducerschicht auf einen Endbereich der Leiterbahn
c) Aufbringen zumindest einer biologischen Komponente auf die Transducerschicht,
d) Bedecken zumindest der Transducerschicht und eines Umgebungsbereiches mit einer mit dem Träger verschweißbaren Deckmembran,
e) Verschweißen der Deckmembran mit dem Träger in den von der Transducerschicht und der biologischen Komponente nicht beanspruchten Bereichen, wobei die Verschweißung im Bereich der Leiterbahn unterbrochen wird.

9. Verfahren zur Herstellung eines planaren Sensors zum Erfassen eines chemischen Parameters einer Probe, **gekennzeichnet durch** folgende Schritte:
a) Aufbringen einer Leiterbahn auf zumindest einen Teilbereich der Oberfläche eines Trägers,
b) Aufbringen eines polymeren Matrixmaterials auf einen Endbereich der Leiterbahn,
c) Immobilisierung eines potentiometrischen oder amperometrischen Transducers und einer biologischen Komponente auf bzw. im polymeren Matrixmaterial,
d) Bedecken zumindest der Transducerschicht und eines Umgebungsbereiches mit einer mit dem Träger verschweiß-baren Deckmembran,
e) Verschweißen der Deckmembran mit dem Träger in den von der Transducerschicht und der biologischen Komponente nicht beanspruchten Bereichen, wobei die Verschweißung im Bereich der Leiterbahn unterbrochen wird.

10. Verfahren nach Anspruch 8 oder 9, **dadurch gekennzeichnet,** daß die Deckmembran von einem Band abgerollt, über die Oberfläche des Trägers gespannt und mit Hilfe eines Heißprägestempels mit dem Träger thermisch verschweißt wird.

11. Verfahren nach Anspruch 8 oder 9, **dadurch gekennzeichnet**, daß die Deckmembran mittels Laserlicht mit dem Träger thermisch verschweißt wird.

12. Verfahren nach Anspruch 8 oder 9, **dadurch gekennzeichnet,** daß in Schritt e) eine flächige Verschweißung durchgeführt wird.

13. Verfahren nach Anspruch 8 oder 9, **dadurch gekennzeichnet**, daß in Schritt e) eine punktförmige Verschweißung durchgeführt wird.

## Claims

1. Planar sensor for determining a chemical parameter of a sample, comprising a substrate (21) whose surface is at least partly plane and is provided with at least one potentiometric or amperometric and, possibly, an optical transducer, and one or more biological components, and a cover membrane (30) on the side facing the sample, - the transducer and the biological component being provided on the surface of the substrate (21), or at least part of the surface, as a sensor spot (22, 25, 26, 27, 28, 29), and the cover membrane (30) surrounding this sensor spot and being welded to the substrate, wherein those sensor spots (22, 25, 26, 27) that comprise a potentiometric or amperometric transducer are in contact with a strip conductor (31) attached to the plane surface of the substrate (21), the seal of the cover membrane (30) being interrupted where the conducting strips (31) lead away from the sensor spots (22, 25, 26, 27).

2. Sensor according to claim 1 with an amperometric arrangement, wherein at least one sensor spot is configured as a working electrode (22, 25, 26), and wherein at least one reference- (23) and/or counterelectrode (24) is provided on the surface of the substrate (21).

3. Sensor according to claim 2, wherein the cover membrane (30) covers the reference-(23) and/or counterelectrode (24) and is sealed around these electrodes (23, 24) to the substrate (21), the seal being interrupted in the area of the strip conductors (31) leading away from the electrodes (23, 24).

4. Sensor according to claim 1 with a potentiometric arrangement, wherein at least one sensor spot is configured as a working electrode (27), and, possibly, at least one reference electrode (28) is provided on the surface of the substrate (21).

5. Sensor according to any of claims 2 to 4, wherein an additional sensor spot (29) with an optical transducer is provided, which has an indicator layer (35) with an optical indicator, and at least one layer (33, 36) with the biological component.

6. Sensor according to any of claims 2 to 4, wherein an additional sensor spot (29) with a polymer matrix (38) is provided, in or on which the optical indicator and the biological component are immobilized either chemically or physically.

7. Sensor according to any of claims 1 to 6, wherein at least two sensor spots are provided, one of them serving as a working electrode (22, 25, 26, 27) and containing an active biological component, and the other one as a comparison electrode (25) containing the same component in a deactivated state.

8. Method of producing a planar sensor for determining a chemical parameter of a sample, wherein the following steps are performed:
a) attaching a strip conductor to at least part of the surface of a substrate,
b) applying a potentiometric or amperometric transducer layer onto one end of the strip conductor,
c) affixing at least one biological component to the transducer layer.
d) covering at least the transducer layer and a surrounding area with a cover membrane to be sealed to the substrate,
e) sealing the cover membrane to the substrate in areas not covered by the transducer layer and the biological substrate, the seal being interrupted in the area of the strip conductor.

9. Method of producing a planar sensor for determining a chemical parameter of a sample, wherein the following steps are performed:
a) attaching a strip conductor to at least part of the surface of a substrate,
b) applying a polymer matrix on one end of the strip conductor,
c) immobilizing a potentiometric or amperometric transducer and the biological component on or in the polymer matrix,
d) covering at least the transducer layer and a surrounding area with a cover membrane to be sealed to the substrate,
e) sealing the cover membrane to the substrate in areas not covered by the transducer layer and the biological substrate, the seal being interrupted in the area of the strip conductor.

10. Method according to claim 8 or 9, wherein the cover membrane is unwound from a roll of tape, stretched over the surface of the substrate and thermally welded to the substrate by means of a heat stamp.

11. Method according to claim 8 or 9, wherein the cover membrane is thermally welded to the substrate by means of a laser technique.

12. Method according to claim 8 or 9, wherein the seal obtained in step (e) is continuous.

13. Method according to claim 8 or 9, wherein the seal obtained in step (e) is in individual spots.

## Revendications

1. Capteur planaire pour la détection d'un paramètre chimique d'un échantillon, constitué d'un support (21) à surface au moins partiellement plane, présentant au moins un transducteur potentiométrique ou ampérométrique ainsi que, le cas échéant, un transducteur optique et au moins un composant biologique, ainsi qu'avec une membrane de recouvrement (30) montée côté échantillon, le transducteur et le composant biologique se présentant dans au moins une zone partielle sur la surface du support (21) sous la forme de ce que l'on appelle un point capteur (22, 25, 26, 27, 28, 29), et la membrane de recouvrement (30) étant soudée au support (21) tout autour du point capteur,
caractérisé en ce que
les points capteurs (22, 25, 26, 27) qui présentent un transducteur potentiométrique ou ampérométrique, sont mis en contact avec une piste conductrice (31) montée sur la surface plane du support (21), le soudage de la membrane de recouvrement (30) étant interrompu dans la zone des pistes conductrices (31) partant du point capteur (22, 25, 26, 27).

2. Capteur selon la revendication 1, avec un dispositif de mesure ampéremétrique,
caractérisé en ce qu'
au moins un point capteur est réalisé sous la forme d'électrode de travail (22, 25, 26) et, en outre, au moins une électrode de référence (23) et/ou une contre-électrode (24) sont disposées sur la surface du support (21).

3. Capteur selon la revendication 2,
caractérisé en ce que
la membrane de recouvrement (30), l'électrode de référence (23) et/ou la contre-électrode (24) sont recouvertes et soudées au support (21) tout autour de ces électrodes (23, 24), le soudage étant interrompu dans la zone des pistes conductrices (31) partant des électrodes (23, 24).

4. Capteur selon la revendication 1, avec un dispositif de mesure potentiométrique,
caractérisé en ce qu'
au moins un point capteur est réalisé sous la forme d'électrode de travail (27) et, le cas échéant, au moins une électrode de référence (28) est disposée sur la surface du support (21).

5. Capteur selon l'une des revendications 2 à 4,
caractérisé en ce qu'
il est prévu un point capteur (29) supplémentaire avec un transducteur optique, qui présente une couche indicatrice (35) dotée d'un indicateur optique, ainsi qu'au moins une couche (33, 36) comportant le composant biologique.

6. Capteur selon l'une des revendications 2 à 4,
caractérisé en ce qu'
il est prévu un point capteur (29) supplémentaire doté d'une matrice en polymère (38), dans laquelle ou sur laquelle l'indicateur optique et le composant biologique se présentent, immobilisés chimiquement ou physiquement.

7. Capteur selon les revendications 1 à 6,
caractérisé en ce qu'
au moins deux points capteurs sont prévus, un point capteur contenant un composant biologique actif, à titre d'électrodes de travail (22, 25, 26, 27), et l'autre, réalisé sous la forme d'une électrode de comparaison (25) présentant le même composant, inactivé.

8. Procédé de fabrication d'un capteur planaire pour la détection d'un paramètre chimique d'un échantillon,
caractérisé par les étapes ci-après :
a) application d'une piste conductrice sur au moins une zone partielle de la surface d'un support,
b) application d'une couche transductrice potentiométrique ou ampéremétrique, sur une zone d'extrémité de la piste conductrice,
c) application d'au moins un composant biologique sur la couche transductrice,
d) couverture d'au moins la couche transductrice et d'une zone environnante avec une membrane de recouvrement soudable au support,
e) soudure de la membrane de recouvrement au support dans les zones non sollicitées par la couche transductrice et le composant biologique, le soudage étant interrompu dans la zone de la piste conductrice.

9. Procédé de fabrication d'un capteur plan pour détecter un paramètre chimique d'un échantillon,
caractérisé par les étapes ci-après :
a) application d'une piste conductrice sur au moins une zone partielle de la surface d'un support,
b) application d'un matériau de matrice polymère sur une zone d'extrémité de la piste conductrice,
c) immobilisation d'un transducteur potentiométrique ou ampéreométrique et d'un composant biologique sur, respectivement dans, le matériau de matrice polymère,
d) couverture d'au moins la couche transductrice et d'une zone environnante avec une membrane de recouvrement soudable au support,
e) soudure de la membrane de recouvrement au support dans les zones non sollicitées par la couche transductrice et le composant biologique, le soudage étant interrompu dans la zone de la piste conductrice.

10. Procédé selon la revendication 8 ou 9,
caractérisé en ce que
la membrane de recouvrement est déroulée d'une bande, est tendue sur la surface du support, et est soudée thermiquement au support à l'aide d'un poinçon de formage à chaud.

11. Procédé selon la revendication 8 ou 9,
caractérisé en ce que
la membrane de recouvrement est soudée thermiquement au support par une lumière laser.

12. Procédé selon la revendication 8 ou 9,
caractérisé en ce qu'
on effectue à l'étape e) un soudage de surface.

13. Procédé selon la revendication 8 ou 9,
caractérisé en ce qu'
on effectue à l'étape e) un soudage par points.
